(19) Europäisches Patentamt
European Patent Office
Office européen des brevets

(11) EP 4 620 517 A1

(12) EUROPEAN PATENT APPLICATION

(43) Date of publication:
24.09.2025 Bulletin 2025/39

(51) International Patent Classification (IPC):
*A61N 1/39* $^{(2006.01)}$

(21) Application number: 24164308.9

(22) Date of filing: 18.03.2024

(52) Cooperative Patent Classification (CPC):
A61N 1/3956; A61N 1/3987

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(71) Applicant: BIOTRONIK SE & Co. KG
12359 Berlin (DE)

(72) Inventors:
• Weiss, Ingo
12435 Berlin (DE)
• Ciesla, Catharina-Sophie
12167 Berlin (DE)

(74) Representative: Biotronik Corporate Services SE
Corporate Intellectual Property
Sieversufer 7-9
12359 Berlin (DE)

(54) **CARDIAC STIMULATION ARRANGEMENT**

(57) The invention relates to an arrangement (200) comprising a non-transvenous implantable cardioverter-defibrillator device (210) for stimulating a human or animal heart, the non-transvenous implantable cardioverter-defibrillator device (210) comprising a stimulation unit (270) for emitting stimulation pulses (140) externally to the heart; and a programming device (220) operatively coupled to the non-transvenous implantable cardioverter-defibrillator device (210); wherein the arrangement is capable to predict an expected time of occurrence of a future T wave $(B_n)$ by determining the future T wave $(B_n)$ on the basis of a function using first coefficients $(a_i)$, second coefficients $(c_i)$ and/or third coefficients $(d_i)$.

FIG. 2A

**Description**

The present invention relates to an arrangement comprising a non-transvenous implantable cardioverter-defibrillator device and a programming device for this non-transvenous implantable cardioverter-defibrillator device according to the preamble of claim 1, as well as to a processing method according to the preamble of claim 14 and to a non-transvenous implantable cardioverter-defibrillator device according to the preamble of claim 15.

A non-transvenous implantable cardioverter-defibrillator device generally is designed for implantation external to a patient's heart. A non-transvenous implantable cardioverter-defibrillator device, in short non-transvenous ICD, comprises a stimulation unit and a processor, and at least one lead comprising a shock electrode for emitting an electrical shock pulse externally to a patient's heart. The lead is connected to the stimulation unit. The stimulation unit forms part of a generator device that may, for example, be implanted subcutaneously in a patient. The lead, in a connected state, extends from the generator device, the lead being implanted such that it fully rests outside of the patient's heart. The lead may for example extend from the generator device towards a location in the region of the patient's sternum, the shock electrode hence being placed outside of the patient's heart for emitting an electrical shock pulse at a location external to the patient's heart.

The term "non-transvenous" in this respect in particular shall express that the lead of the non-transvenous implantable cardioverter-defibrillator device does not extend transvenously into the heart, but fully rests outside of the patient's heart.

The non-transvenous implantable cardioverter-defibrillator device in particular is designed for emitting electrical shocks in case life-threatening arrhythmias of a patient's heart are detected. By means of an electrical shock, a defibrillation shall be achieved in order to reset the cardiac rhythm back to a normal state.

In some instances, T wave shocks are applied with a fixed, manually predetermined delay of the shock with respect to the previous QRS complex in the electrocardiogram (Barold, H. S., & Wharton, J. M. (1997). Ventricular fibrillation resulting from synchronized internal atrial defibrillation in a patient with ventricular preexcitation. Journal of Cardiovascular Electrophysiology, 8(4), 436-440). However, this approach does not consider possible variations in the cardiac rhythm of the patient.

When operating a non-transvenous implantable cardioversion defibrillation device, it is helpful to estimate the time point of the next T wave of the cardiac rhythm of the patient. Typically, shock pulses for achieving a cardioversion are applied outside the T wave. If the cardioversion would be performed during the T wave, there is a high risk of inducing ventricular fibrillation by the cardioversion shock pulses.

In some instances, such ventricular fibrillation of the patient's heart is desired. For this purpose, sequences of pulse bursts are emitted by the shock electrode. As such pulse bursts may be emitted over a duration of several seconds and may be delivered with substantial energy, such pulse bursts may cause a significant stress on a patient and its muscular system, causing potentially postoperative pain to a patient. R-wave synchronized inducing of ventricular fibrillation has been described by Wylie Jr. et al. (Wylie Jr, J. V., Essebag, V., Reynolds, M. R., & Josephson, M. E. (2009). Inducibility of Atrial Fibrillation with a Synchronized External Low Energy Shock Post-Pulmonary Vein Isolation Predicts Recurrent Atrial Fibrillation. Journal of Cardiovascular Electrophysiology, 20(1), 29-36). however, and exact knowledge on the time of occurrence of the T wave increases the efficacy of such pulse bursts.

In some instances, an electric (or galvanically coupled) communication between different implantable or implanted medical devices is desired. The risk of ventricular fibrillation is significantly reduced if any electric communication pulses are emitted by such devices only outside the T waves of the patient's cardiac rhythm.

Thus, there is a general need of an exact estimation of the next T wave in the patient's cardiac rhythm for these different applications of emitting electric pulses during or outside the T wave.

US 2003/0195569 A1 discloses a method for determining a cardiac shock strength, for example a programmed first therapeutic shock strength of an implantable cardioverter-defibrillator, including sensing a change in a T wave of an electrogram with respect to time, delivering a test shock by delivering a test shock at a test shock strength and at a test shock time relating to a maximum of a first derivative of the T wave with respect to time, and sensing for cardiac fibrillation. The implantable cardioverter-defibrillator herein is designed for a transvenous implantation.

Another transvenous cardioverter-defibrillator device is disclosed for example in US 7,386,342.

It is an object of the present invention to provide an arrangement comprising a non-transvenous implantable cardioverter-defibrillator device, a method for operating such an arrangement and a non-transvenous implantable ICD, which allow for a more exact estimation of the occurrence of the T wave in a patient's natural cardiac rhythm without the need for an additional background pacing.

This object is achieved with an arrangement comprising a non-transvenous implantable cardioverter-defibrillator device and a programming device operatively coupled to the non-transvenous implantable cardioverter-defibrillator device. The non-transvenous implantable cardioverter-defibrillator device (non-transvenous ICD) serves for stimulating a human or animal heart. It comprises a stimulation unit for emitting stimulation pulses externally to a patient's heart.

The programming device comprises a first processor and a first memory unit. The first memory unit comprises first computer-readable code that causes the first processor to perform the steps explained in the following when executed on the first processor.

In a first step, cardiac cycle data based on an analysis of first electrocardiogram signals indicative of a natural cardiac activity of the heart is obtained from the first memory unit. In an embodiment, the first electrocardiogram signals may be received before from the non-transvenous ICD or an extracardiac device, may then be analysed by the first processor to obtain the cardiac cycle data and stored in the first memory unit, or the cardiac cycle date may be received before directly by the programmer and stored in the first memory unit. Thus, the arrangement does not require inducing a synthetic cardiac rhythm to the patient to whom the non-transvenous ICD is implanted. Rather, the arrangement bases its algorithm on the natural cardiac activity of the patient.

Afterwards, a function for predicting an expected time of occurrence of a future T wave (Bn) is evaluated using the cardiac cycle data to determine at least one of a plurality of first coefficients ($a_i$), a plurality of second coefficients ($c_i$) and/or a plurality of third coefficients ($d_i$). As the person skilled in the art is well aware of, the T wave represents the repolarization of the cardiac ventricles and occurs after a cardiac depolarization (represented in an electrocardiogram by the so-called QRS complex).

The function comprises at least one of i) a sum of powers of a first interval (AA) between two subsequent ventricular contraction events, wherein each power of the first interval (AA) is multiplied by a first coefficient ($a_i$), ii) a sum of powers of a second interval (C) between a ventricular contraction event and a subsequent T wave, wherein each power of the second interval (C) is multiplied by a second coefficient ($c_i$), and iii) a sum of powers of a third interval (D) between a T wave and a subsequent ventricular contraction event, wherein each power of the third interval (D) is multiplied by a third coefficient ($d_i$). The first coefficients ($a_i$), the second coefficients ($c_i$) and/or the third coefficients ($d_i$) are then transmitted to the non-transvenous ICD.

The non-transvenous ICD comprises a second processor and a second memory unit, wherein the second memory unit comprises a second computer-readable code that causes the second processor to perform the steps explained in the following when executed on the second processor.

First, second electrocardiogram signals indicative for a natural cardiac activity of the heart are received. In an embodiment, the non-transvenous ICD may comprise a sensing unit for sensing the second electrocardiogram signals. In another embodiment, the second electrocardiogram signals may be sensed by an extracardiac device and send to the non-transvenous ICD. Afterwards, the electrocardiogram signals are processed to predict an expected time of occurrence of a future T wave. In this context, the expected time of occurrence of thefuture T wave is calculated by the function using the first coefficients ($a_i$), the second coefficients ($c_i$) and/or the third coefficients ($d_i$).

The special advantage of the above described arrangement separates the calculation steps to be performed in order to predict an expected time of occurrence of a future T wave such that computing-intensive calculations are performed in the programming device and less computing-intensive calculations are performed by the non-transvenous ICD. In doing so, the battery capacity of the non-transvenous ICD is not used to a relevant extent for estimating a future T wave.

In an embodiment, the first computer-readable code may further cause the first processor to perform the following steps when executed on the first processor: optimizing the first coefficients ($a_i$), the second coefficients ($c_i$) and/or the third coefficients ($d_i$) with an optimizing algorithm to obtain optimized first coefficients, optimized second coefficients and/or optimized third coefficients before transmitting to the non-transvenous implantable cardioverter-defibrillator device. Here and in the following description, the sum of powers of the first interval (AA) is meant to be a sum of $AA^i$ terms, the sum of powers of the second interval (C) is meant to be a sum of $C^i$ terms, and the sum of powers of the third interval (D) is meant to be a sum of $D^i$ terms, where i is a natural number. By way of an additional optimization step, an estimation of the future T wave is performed in a very exact manner so that the non-transvenous ICD can emit stimulation pulses either during the predicted T wave or outside the predicted T wave, depending on the desired result of the stimulation pulses.

The first coefficients ($a_i$), the second coefficients ($c_i$) and/or the third coefficients ($d_i$) serve for describing the model that in turn describes the cardiac rhythm of the patient. Due to the optimization of these coefficients, the model description becomes more exact so that the time of occurrence of a future T wave can be predicted much better than according to prior art techniques.

While prior art non-transvenous ICDs often stimulate the patient's heart to achieve a constant (yet artificial) cardiac rate being somewhat faster than the intrinsic cardiac rhythm, the presently claimed arrangement exactly predicts the expected time of occurrence of a future T wave in the natural cardiac rhythm. Thus, no prior cardiac stimulation of the patient's heart is necessary. This reduces the cardiac stress for the patient and also reduces the energy consumption of the non-transvenous ICD with respect to prior art devices.

In an embodiment, at least one of the sum of powers of the first interval (AA) forms a series expansion in powers 0 to p of the first interval (AA), wherein the first coefficients ($a_i$) form expansion coefficients, the sum of powers of the second interval (CC) forms a series expansion in powers 0 to q of the second interval (C), wherein the second coefficients ($c_i$) form expansion coefficients, and the sum of powers of the third interval (D) forms a series expansion in powers 0 to m of the third interval (D), wherein the third coefficients ($d_i$) form expansion coefficients, wherein m, p, q are predetermined natural numbers. In an embodiment, the predetermined natural numbers m, p, q may range from 1 to 10, in particular of from 1 to 3 or from 1 to 4 or from 1 to 5.

In an embodiment, the series expansion in the first interval (AA), the series expansion in the second interval (C) and/or the series expansion in the third interval (D) forms at least a part of a Taylor series.

In an embodiment, the second computer-readable code causes the second processor to generate at least one stimulation pulse emitted by the stimulation unit during the expected time of occurrence of the future T wave ($B_n$). Such a stimulation pulse emitted during the future T wave ($B_n$) typically induces ventricular fibrillation in the patient's heart.

In an embodiment, the second computer-readable code causes the second processor to generate at least one stimulation pulse emitted by the stimulation unit outside (i.e., prior to or after) the expected time of occurrence of the second future T wave ($B_n$). Such a stimulation pulse emitted outside the second future T wave ($B_n$) is typically used for achieving a cardioversion of the patient's heart and for restoring a physiologic cardiac rhythm.

In an embodiment, the programming device triggers the second processor of the non-transvenous implantable cardioverter-defibrillator to generate the at least one stimulation pulse emitted by the stimulation unit.

In an embodiment, the second computer-readable code causes the second processor to emit at least one electric pulse by the stimulation unit or by a communication unit of the non-transvenous ICD outside the expected time of occurrence of the second future T wave. Such electric communication pulses should be typically not emitted during the occurrence of a T wave to avoid any risk of inducing ventricular fibrillation. Rather, by emitting electric communication pulses outside the predicted time of occurrence of the T wave, the risk of undesired cardiac effects due to the emitted electric pulses is significantly reduced.

In an embodiment, the optimized first coefficients assigned to elder intervals between two subsequent ventricular contraction events have smaller absolute values than the optimized first coefficients assigned to younger intervals between two subsequent ventricular contraction events. Expressed in other words, older intervals are less strongly weighted than younger intervals. By such different strong weighting of intervals depending on their age with respect to the T wave to be predicted, variations of the cardiac rhythm over time can be well compensated for. It is then assumed that such variations leading to a different cardiac rhythm and occurring more recently prior to the predicted T wave are in fact more relevant for an exact prediction of the time of occurrence of this T wave.

Alternatively or additionally, the optimized second coefficients assigned to elder intervals between a ventricular contraction event and a subsequent T wave have smaller absolute values than the optimized second coefficients assigned to younger intervals between a ventricular contraction event and a subsequent T wave. Also this stronger weighting of more recent cardiac contractions serves for a higher accuracy of the predicted type of occurrence of the future T wave.

Alternatively or additionally, the optimized third coefficients assigned to elder intervals between a T wave and a subsequent ventricular contraction event have smaller absolute values than the optimized third coefficients assigned to younger intervals between a T wave and a subsequent ventricular contraction event. Likewise, this stronger weighting of more recent cardiac events with respect to the future T wave serves for a higher accuracy of prediction of the time of occurrence of this T wave.

A different strong weighting of individual cardiac contraction events can also be achieved with a further mode of optimization of the individual coefficients according to an embodiment of the presently claimed arrangement. According to this embodiment, at least one of the absolute values of the optimized first coefficients, the absolute values of the optimized second coefficients, and the absolutes value of the optimized third coefficients decreases monotonically at least section-wise with increasing temporal distance between the second future T wave and the respective interval to which the individual coefficients of the optimized first coefficients, the optimized second coefficients, and/or the optimized third coefficients are assigned, respectively. By such a time-dependent weighting of the optimized individual coefficients, a stronger weighting of younger cardiac events (with respect to the future T wave, the expected time of occurrence is to be predicted) than of elder cardiac events is achieved. Due to the monotonic decrease of the coefficients, each younger cardiac event is stronger weighted than any of the preceding elder cardiac events.

In an embodiment, the cardiac cycle data comprises at least a length of the second interval (C) of a first cardiac cycle and at least one of a length of the first interval (AA) of the second cardiac cycle, a length of the second interval (C) of the second cardiac cycle, and a length of the third interval (D) of the second cardiac cycle, wherein the second cardiac cycle precedes the first cardiac cycle.

In an embodiment, the function corresponds to the following equation comprising a Taylor series:

$$C_n = \sum_{i=0}^{m_1} d_{i1}D_{n-1}^i + \cdots + \sum_{i=0}^{m_j} d_{ij}D_{n-j}^i + \sum_{i=0}^{p_1} a_{i1}AA_{n-1}^i + \cdots + \sum_{i=0}^{p_k} a_{ik}AA_{n-k}^i$$

$$+ \sum_{i=0}^{q_1} c_{i1}C_{n-1}^i + \cdots + \sum_{i=0}^{q_l} c_{il}C_{n-l}^i$$

In this context,

| | |
|---|---|
| $C_n$ | denotes the second interval in a first cardiac cycle; |
| $a_{i1}...a_{ik}$ | denote the first coefficients or the optimized first coefficients; |
| $c_{i1}...c_{il}$ | denote the second coefficients or the optimized second coefficients; |
| $d_{i1}...d_{ij}$ | denote the third coefficients or the optimized third coefficients; |
| $AA_{n-1}...AA_{n-k}$ | denote the first intervals in a second cardiac cycle and preceding cardiac cycles prior to the first cardiac cycle; |
| $C_{n-1}...C_{n-l}$ | denote the second intervals in the second cardiac cycle and preceding cardiac cycles prior to the first cardiac cycle; |
| $D_{n-1}...D_{n-j}$ | denote the third intervals of the second cardiac cycle and preceding cardiac cycles prior to the first cardiac cycle; |
| j, k, l | denote independently from each other a number of cardiac cycles preceding the first cycle and may take values in a range from 1 to 10; |
| m, p, q | denote the largest powers of the first, second and third intervals in the series expansions and are programmable independently from each other. m, p, q may take values in a range from 1 to 5, in particular from 1 to 3. |

In an embodiment, the first computer-readable code causes the first processor to perform the following step and/or in that the second computer-readable code causes the second processor to perform the following step: performing pattern-identification calculations resulting in pattern-recognition information and storing the pattern-recognition information in the first or the second memory unit, respectively.Performing the pattern-identification calculations by the first processor of the programmer has the special advantage that power consuming calculation steps need not to be performed within the non-transvenous ICD. Such an approach saves battery capacity of the non-transvenous ICD.

In an embodiment, the first computer-readable code causes the first processor to perform the following step and/or in that the second computer-readable code causes the second processor to perform the following step: applying a pattern-tracking algorithm on the electrocardiogram signals to identify cardiac events in the first or the second electrocardiogram signals. Such pattern-tracking algorithms allow an identification of cardiac signals on the basis of previously identified patterns with less effort than in case of signal identification without such pattern-tracking algorithm. A device or algorithm for correlation waveform analysis (CWA) is particularly appropriate to apply pattern tracking. Pattern-tracking algorithms can be particularly appropriate applied on the slope of a curve representing electrocardiogram signals.

In an embodiment, the pattern-tracking algorithm involves correlating signals of the electrocardiogram signals with the stored pattern-recognition information. , wherein correlating signals of the electrocardiogram signals, in particular, involves a convolution of the electrocardiogram signals. In doing so, an easy identification of specific patterns and thus cardiac events can be accomplished by the non-transvenous ICD with outperforming complex and/or power consuming calculations. This embodiment further makes use of the general concept of aspects of the present invention that is directed to split-off calculations to be performed by the arrangement such that power-consuming calculations are done by the programming device and less power-consuming calculations are performed within the non-transvenous ICD. This approach results in very exact results while requiring only little battery capacity of the non-transvenous ICD.

In an embodiment, correlating the signals involves a convolution of the electrocardiogram signals with the pattern. A correlation by convolution typically is less time and power consuming than other correlations.

In an embodiment, the pattern-tracking algorithm is only repeatedly applied to the electrocardiogram signals in time periods in which the cardiac event to be detected is expected to occur. By avoiding a continuous search for the respective pattern, the required computing capacity and thus the required power consumption is significantly reduced. Typically, a predeterminable safety distance is applied prior to the estimated time of occurrence of the cardiac event to be detected. Then, a safe detection of this cardiac event is still possible even without continuous search for specific patterns within the electrocardiogram signals.

In an embodiment, the first computer-readable code causes the first processor to optimize the first coefficients ($a_i$), the second coefficients ($c_i$) and/or the third coefficients ($d_i$) by a series of steps. According to a first step, a factual time of occurrence of the future T wave ($B_n$) is measured. According to a second step, a difference between the factual time of occurrence of the future T wave ($B_n$) and the expected time of occurrence of the future T wave ($B_n$) is determined. According to a third step, the determined difference is iteratively reduced until it falls below a predeterminable threshold. In this context, the predeterminable threshold lies in a range of from 10 ms to 100 ms, in particular of from 20 ms to 90 ms, in particular of from 30 ms to 80 ms, in particular of from 40 ms to 70 ms, in particular of from 50 ms to 60 ms. In doing so, the accuracy of determination of the expected time of occurrence of the future T wave can be adjusted to the respective needs. Typically, a high accuracy can be achieved by a longer (and thus more power consuming) iterative reducing of the difference between the expected time of occurrence of the future T wave ($B_n$) and the factual time of occurrence of the future T wave ($B_n$). Since the electric power available for the programming device is generally not limited, the desired accuracy is mainly dependent on the computing time to be spent for the optimization process.

In an embodiment, the electrocardiogram signals received by the programming device are provided by the non-transvenous implantable cardioverter-defibrillator device. Then, it is not necessary to otherwise provide electrocardiogram signals to the programming device. In an alternative embodiment, the electrocardiogram signals received by the programming device are provided by an electrode applied to a body surface of the patient. Then, the programming device and the non-transvenous ICD use different electrodes for obtaining information on the cardiac rhythm of the patient.

In an embodiment, the programming device considers all intervals being older than n-1, (i.e. e.g. the cardiac device considers cardiac cycle data of cardiac cycles older than the second cardiac cycle) and optimizes the coefficients based on these values. The non-transvenous ICD then only calculates the expected time of occurrence of the future T wave $B_n$ (e.g. in the first cardiac cycle) by additionally considering the most recent interval, i.e., the n-1 interval (which means e.g. by using the cardiac cycle data of the second cardiac cycle). In doing so, the time necessary for transferring the optimized coefficients from the programming device to the non-transvenous ICD is compensated for. Sometimes, the transfer takes longer than the determination of the expected time of occurrence of the future T wave within the non-transvenous ICD. However, it is necessary to determine the expected time of occurrence of the future T wave very quick since the stimulation pulse needs to be applied in real time.

In an embodiment, for predicting the time of occurrence of a further future T wave $B_{n+1}$ the programmer may consider cardiac cycle data of the second cardiac cycle n-1 and then additionally considers the previously predicted intervall $C_n$. In this way, the programmer is able to predict the expected time of occurrence of the future T wave further ahead into the future.

According to an aspect of the present invention, a processing method for determining an expected time of occurrence of a future T wave ($B_n$) in a cardiac cycle of a human or animal heart is provided, wherein the method is carried out by a processor of a medical device and comprises the following steps: obtaining cardiac cycle data based on an analysis of first electrocardiogram signals indicative of a natural cardiac activity of the heart; evaluating a function for predicting an expected time of occurrence of a future T wave ($B_n$) using the cardiac cycle data to determine at least one of a plurality of first coefficients ($a_i$), a plurality of second coefficients ($c_i$) and a plurality of third coefficients ($d_i$); wherein the function comprises at least one of i) a sum of powers of a first interval (AA) between two subsequent ventricular contraction events, wherein each power of the first interval (AA) is multiplied by a first coefficient ($a_i$), ii) a sum of powers of a second interval (C) between a ventricular contraction event and a subsequent T wave, wherein each power of the second interval (C) is multiplied by a second coefficient ($c_i$), and iii) a sum of powers of a third interval (D) between a T wave and a subsequent ventricular contraction event, wherein each power of the third interval (D) is multiplied by a third coefficient ($d_i$), receiving second electrocardiogram signals indicative of a natural cardiac activity of the heart; processing the second electrocardiogram signals to predict an expected time of occurrence of a future T wave ($B_n$), wherein the expected time of occurrence of the future T wave ($B_n$) is calculated by the function using the first coefficients ($a_i$), the second coefficients ($c_i$) and/or the third coefficients ($d_i$).

According to a further apect, the present invention includes a non-transvenous implantable cardioverter-defibrillator device for stimulating a human or animal heart, the non-transvenous implantable cardioverter-defibrillator device comprising a stimulation unit for emitting stimulation pulses externally to the heart, a processor and a memory unit, wherein the memory unit comprises computer-readable code that causes the processor to perform the following steps when executed on the processor: obtaining, from the memory unit, cardiac cycle data based on an analysis of first electrocardiogram signals indicative of a natural cardiac activity of the heart; evaluating a function for predicting an expected time of occurrence of a future T wave ($B_n$) using the cardiac cycle data to determine at least one of a plurality of first coefficients ($a_i$), second coefficients ($c_i$) and/or third coefficients ($d_i$); wherein the function comprises at least one of i) a sum of powers of a first interval (AA) between two subsequent ventricular contraction events, wherein each power of the first interval (AA) is multiplied by a first coefficient ($a_i$), ii) a sum of powers of a second interval (C) between a ventricular contraction event and a subsequent T wave, wherein each power of the second interval (C) is multiplied by a second coefficient ($c_i$), and iii) a sum of powers of a third interval (D) between a T wave and a subsequent ventricular contraction event, wherein each power of the third interval (D) is multiplied by a third coefficient ($d_i$), receiving second electrocardiogram signals indicative of a natural cardiac activity of the heart; processing the second electrocardiogram signals to predict an expected time of occurrence of a future T wave ($B_n$), wherein the expected time of occurrence of the future T wave ($B_n$) is calculated by the function using the first coefficients ($a_i$), the second coefficients ($c_i$) and/or the third coefficients ($d_i$).

All embodiments of the arrangement can be combined in any desired way and can be transferred either individually or in any arbitrary combination to the described method and the describes non-transvenous implantable ICD. Likewise, all embodiments of the described method can be combined in any desired way and can be transferred either individually or in any arbitrary combination to the described arrangement and the describes non-transvenous implantable ICD. Likewise, all embodiments of the described non-transvenous implantable ICD can be combined in any desired way and can be transferred either individually or in any arbitrary combination to the described arrangement or the descrbed method.

Further details of aspects of the present invention will be explained in the following with reference to exemplary embodiments and accompanying Figures. In the Figures:

Figure 1A      shows a schematic depiction of the application of a T wave shock in a transvenous ICD known from prior art;

Figure 1B      shows the application of a cardioversion in a common transvenous ICD known from prior art;

Figure 2A      shows a schematic depiction of a first embodiment of a cardiac stimulation arrangement comprising a non-transvenous ICD;

Figure 2B      shows a schematic depiction of a second embodiment of a cardiac stimulation arrangement comprising a non-transvenous ICD;

Figure 2C      shows a flowchart of an exemplary coefficient optimization process that can be performed by the arrangements shown in Figures 2A and 2B;

Figure 3      exemplarily shows different intervals that can be used for calculating an expected time of occurrence of a future T wave;

Figure 4      exemplarily shows different identification possibilities for identifying and/or analyzing individual signals within an electrocardiogram;

Figures 5A and 5B      schematically illustrate a pattern-tracking mechanism;

Figure 6      schematically illustrates an embodiment of a two-step signal analysis;

Figure 7A      illustrates an embodiment of a function for calculating the expected time of occurrence of a future T wave;

Figure 7B      is a graphic representation of the first sum term of the equation shown in Figure 7A;

Figure 7C      is a graphic representation of the second sum term of the equation shown in Figure 7A; and

Figure 7D      is a graphic representation of the third sum term of the equation shown in Figure 7A.

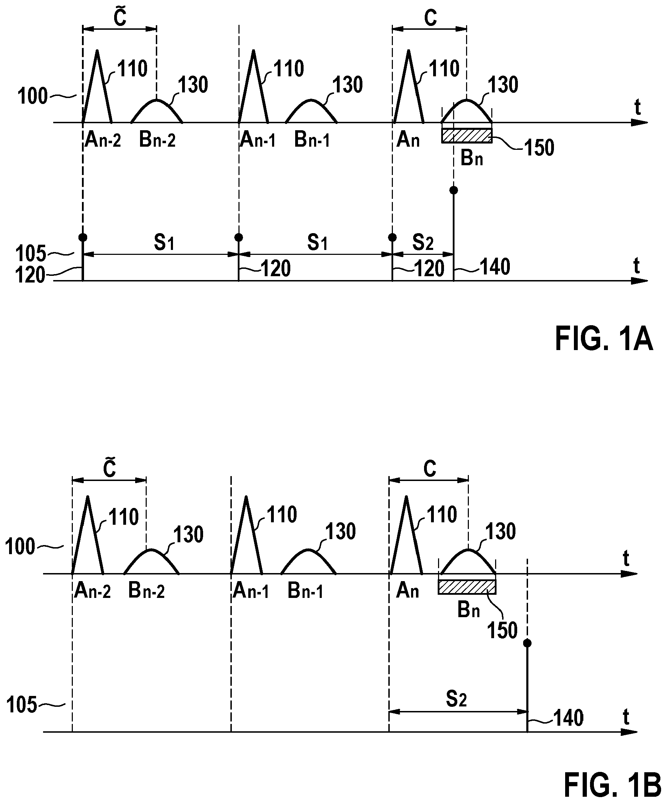

Figure 1A exemplarily shows the application of a T wave shock with a common transvenous ICD according to prior art. On a first measuring channel 100, electrocardiogram signals representing cardiac activity are sensed. "A" events 110 ($A_n$, $A_{n-1}$, $A_{n-2}$; representing cardiac depolarization which can typically best be observed by an R wave of the QRS complex) are typically enforced by a synthetic cardiac rhythm due to regularly applied stimulation pulses 120. These stimulation pulses 120 are applied in a temporal distance $S_1$. These stimulation pulses 120 are emitted on an output channel 105 of the ICD system.

The occurrence of a "B" event 130 (corresponding to a T wave in the electrocardiogram) is typically manually determined. For this purpose, a value $\tilde{C}$ is measured that corresponds to the distance between the "B" event 130 and the preceding "A" event 110. This value $\tilde{C}$ is set as a fixed value C in the transvenous ICD system. The fixed value C can be, e.g., an average value of a plurality of $\tilde{C}$ values.

The time point of emitting a shock pulse 140 is calculated by $S_2 = \kappa * C$. Then, the shock pulse 140 is emitted within the vulnerable window 150 of the ventricles to be stimulated. Consequently, a T wave shock resulting in ventricular fibrillation is applied within the relevant time window.

The enforced stimulation ensures a relatively constant value for C. Such enforced stimulation is, however, an additional stress for the patient in case of non-transvenous ICD systems and is therefore to be avoided.

Figure 1B exemplarily shows a cardioversion process also known from prior art. In this and in all following Figures, similar elements will be denoted with the same numeral reference.

The different "A" events 110 are automatically detected by the ICD. The distance C is again determined by manually measuring the distance $\tilde{C}$ between the "B" event (T wave) 130 and the directly preceding "A" event 110 (R wave of a QRS complex). The time point of emitting a shock pulse 140 is calculated as $S_2 = \kappa * C$ so that the shock pulse 140 is emitted outside the vulnerable window of the ventricles. Then, the shock pulse 140 results in a cardioversion, not in ventricular fibrillation like in case of a T wave shock as illustrated in Figure 1A.

A problem occurs if the intrinsic cardiac rhythm significantly varies since this results in a variation of the correct value of C. Furthermore, the effects of cardiac restitution are to be considered. An acceleration of the cardiac rhythm results in a shortening of a preceding diastolic interval and thus in a shortening of the repolarization duration. Due to this shortening, the calculated value $S_2$ for the shock pulse 140 to be applied outside the vulnerable window of the ventricles can erroneously fall within the vulnerable phase. Likewise, the shock pulse 140 can also be erroneously applied outside the vulnerable window 150 even though it was intended to be applied within the vulnerable window 150 (like in case of Figure 1A).

This illustrates the need for an exact determination of the occurrence of a future T wave, i.e. of the distance C.

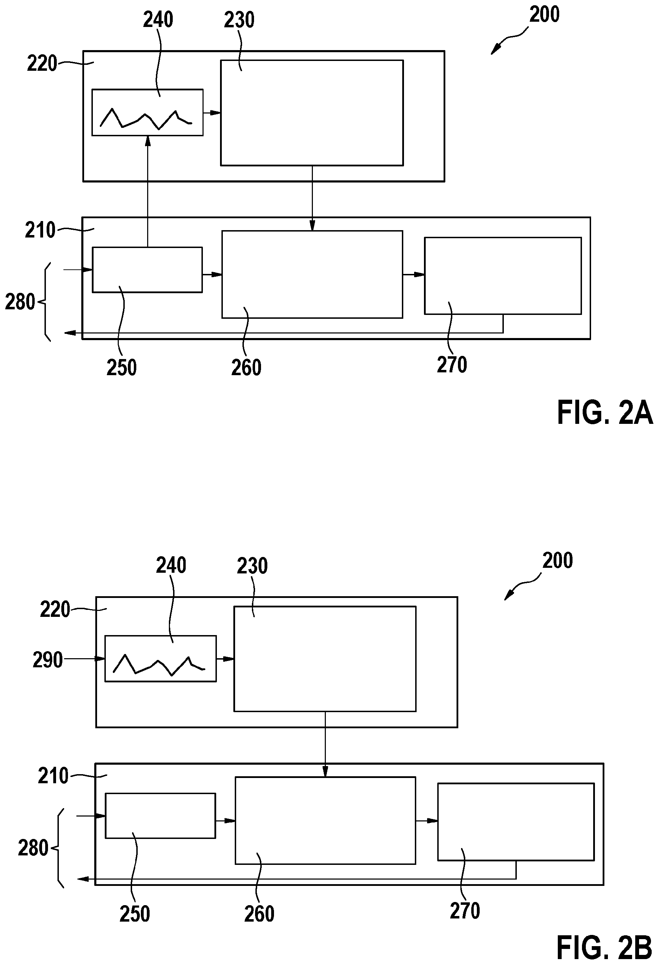

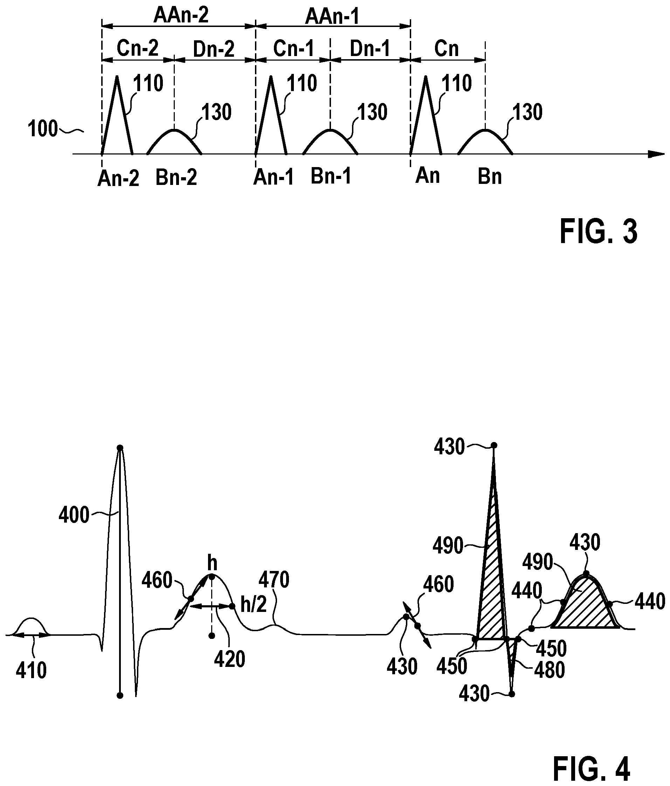

Figure 2A shows an exemplary arrangement 200 comprising a non-transvenous implantable cardioverter-defibrillator device (ICD) 210 and a programming device 220 operatively coupled to the non-transvenous ICD 210. The programming device 220 comprises a first processor 230 that receives, during operation, electrocardiogram signals 240 that are provided by a sensing unit 250 of the non-transvenous ICD 210. The first processor 230 processes the electrocardiogram signals 240. For this purpose, it determines a plurality of "AA" intervals between subsequent "A" events (cf. Figure 3 for more details). Alternatively or additionally, the first processor 230 identifies a plurality of "C" intervals between an "A" event and a subsequent "B" event (cf. Figure 3 for more details). Alternatively or additionally, the first processor 230 identifies a plurality of "D" intervals between a "B" event and a subsequent "A" event (cf. Figure 3 for more details).

The first processor 230 then optimizes individual coefficients that are used to multiply the determined intervals. A set of optimized coefficients results that is transferred in a wireless manner to a second processor 260 of the non-transvenous ICD 210. The second processor 260 receives electrocardiogram signals from the sensing unit 250. It processes the signals with the help of the transferred optimized coefficients. This results in an expected time of occurrence of a second future "B" event, i.e., the second future T wave. A stimulation unit 270 of the non-transvenous ICD 210 is then controlled by the second processor 260 to emit a second stimulation pulse at the calculated expected time of occurrence of the second future T wave. Due to the optimization of the coefficients, the expected time of occurrence of the second future T wave is very exactly determined so that the risk that the second stimulation pulse is emitted outside the expected T wave is significantly reduced with respect to prior art solutions.

The sensing of the electrocardiogram signals by the sensing unit 250 as well as the emission of the stimulation pulse by the stimulation unit 270 is performed via an electrode 280 that is implanted outside the patient's heart (e.g., in the region of the patient's sternum). A housing of the non-transvenous ICD 210 is used as counter electrode.

Figure 2B shows a second embodiment of an arrangement 200 which is very similar to the embodiment shown in Figure 2A. The only difference is that the electrocardiogram signals 240 are not provided by the sensing unit 250 of the non-transvenous ICD 210, but are rather provided to the programming device 220 by electrodes 290 applied on a body surface of the patient to whom the non-transvenous ICD 210 is implanted. All other functions of this embodiment of the arrangement 200 are identical to the functions of the arrangement 200 of Figure 2A so that reference is made to the explanations given above.

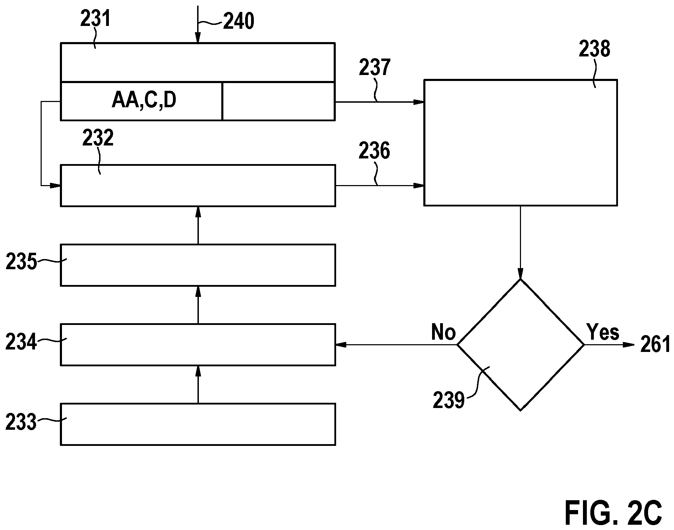

Figure 2C schematically depicts an embodiment of an optimization process for obtaining optimized coefficients. Electrocardiogram signals 240 are provided. A plurality of intervals AA, C, and/or D is determined in a first determination step 231. Based on the determined intervals AA, C and/or D, a model equation 232 serving as the function is determined. For this purpose, initial coefficients $a_0, c_0, d_0$ are provided in a providing step 233. In an optimization step 234, these coefficients are optimized so that initially optimized coefficients a, c, d result in an output step 235. These initially optimized coefficients are then applied to the model equation 232. Further optimization is carried out by comparing an expected time 236 of occurrence of the future "B" event (T wave) and a measured factual time 237 of occurrence of the future "B" event (T wave). This comparison is carried out by calculating 238 the sum of the squared differences between the measured factual time 237 of occurrence of the next "B" event and the expected time 236 of occurrence of the next "B" event.

Afterwards, in a decision step 239, it is determined whether the calculated sum of the squared differences is smaller than a programmable tolerable error threshold. If this is not the case, the steps of optimizing 234, outputting the optimized coefficients 235, applying the output optimized coefficients to the model equation 232 and calculating 238 the sum of the squared differences is iteratively repeated until the resulting sum of squared differences is below the programmable tolerable error threshold. Once this is the case, optimized coefficients 261 are obtained that are then transferred to the second processor 260 of the non-transvenous ICD 210 (cf. Figures 2A and 2B).

It is immediately apparent that the calculating step 238 and the decision step 239 can also be performed on the basis of other measures for a deviation between the expected time 236 of occurrence of the next "B" event and the measured factual time 237 of occurrence of the next "B" event than the squared differences between those values.

Figure 3 illustrates the metrics or intervals determined in the determination step 231 of the signal processing of the electrocardiogram signals measured by 240 or 250 (cf. Figure 2C). "AA" intervals are measured between two subsequent "A" events 110. "C" intervals are measured between a preceding "A" event 110 and a subsequent "B" event 130. Finally, "D" intervals are measured between a preceding "B" event 130 and a subsequent "A" event 110. Apparently, there is an interdependence between "AA" intervals, "C" intervals and "D" intervals. However, the individual intervals cannot necessarily be calculated from each other since the cardiac rhythm is subject to physiologic (and sometimes to non-physiologic) variations. Therefore, "AA" intervals, "C" intervals, and/or "D" intervals generally contain different timing information on the cardiac rhythm and can be used together or alternatively for an exact determination of the expected time of occurrence of the next future "B" event 130.

Figure 4 illustrates various possibilities of signal identification during the processing of the electrocardiogram signals. A signal height 400, a signal width 410, and a full width at half maximum 420 are particular appropriate measures for identifying signals in the electrocardiogram. In addition, the absolute value of extrema 430, inflection points 440, and/or zero crossings 450 are appropriate measures. Likewise, a slope 460, a curvature 470 as well as change of signs 480 or areas 490 bearing a positive sign are appropriate measures for signal identification.

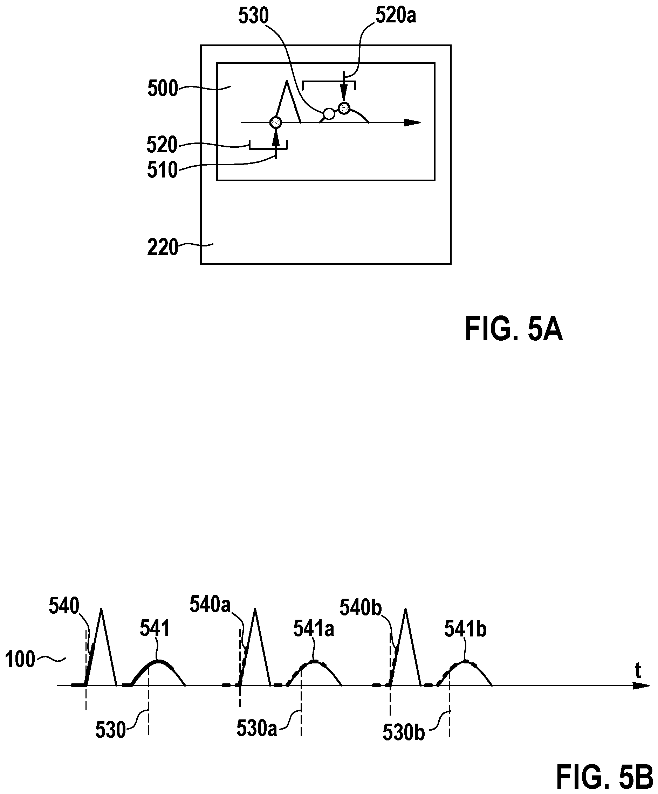

Figure 5A shows an embodiment of a programming device 220. This programming device 220 bears an interactive display 500 that enables the determination of patterns for cardiac depolarization and/or cardiac repolarization. To give an example, these patterns can be marked with a cursor, a mouse or a touchscreen and stored as pattern marker 510. Signal sections in a window 520 around the pattern marker 510 are then used as target pattern for pattern tracking. It is also possible to additionally mark a characteristic point or section within the pattern marker 510 that may have a fixed relative position to the pattern to be identified. In case of a T wave shock, the target pattern for the T wave can be anchored at a central point 520a within the target pattern. Nonetheless, it is possible to emit the T wave shock with a relative offset to this central point 520a. In the embodiment of Figure 5A, a T wave shock emission marker 530 marks the time point at which the T wave shock is to be applied.

Figure 5B shows further details of the pattern tracking applied by the programming device 220 of Figure 5A (and a non-transvenous ICD coupled with this programming device 220, but not illustrated in Figures 5A and 5B).

A first target pattern 540 and a second target pattern 541 that have been determined once, can be easily identified in subsequent cardiac events by evaluating only discrete signal portions 540a, 541a, 540b, and 541b in those subsequent electrocardiogram signals. In the embodiment shown in Figure 5B, this is done with a correlation waveform analysis (CWA). For such analysis, a correlation coefficient is calculated between the first target pattern 540 and the electrocardiogram signals in an evaluation window covering subsequent cardiac signals. If this correlation coefficient is higher than a predetermined threshold, the respective signal is identified as a kind of signal to which the first target pattern 540 has been assigned. This pattern tracking algorithm can be applied both in the programming device 220 and in the coupled non-transvenous ICD 210 (cf. Figures 2A and 2B). Preferably, the pattern-tracking algorithm is performed in the non-transvenous ICD 210. Based on the additional signals identified within the electrocardiogram, subsequent T wave shock emission markers 530a and 530b are determined in the same way as the first T wave shock emission marker 530 has been determined.

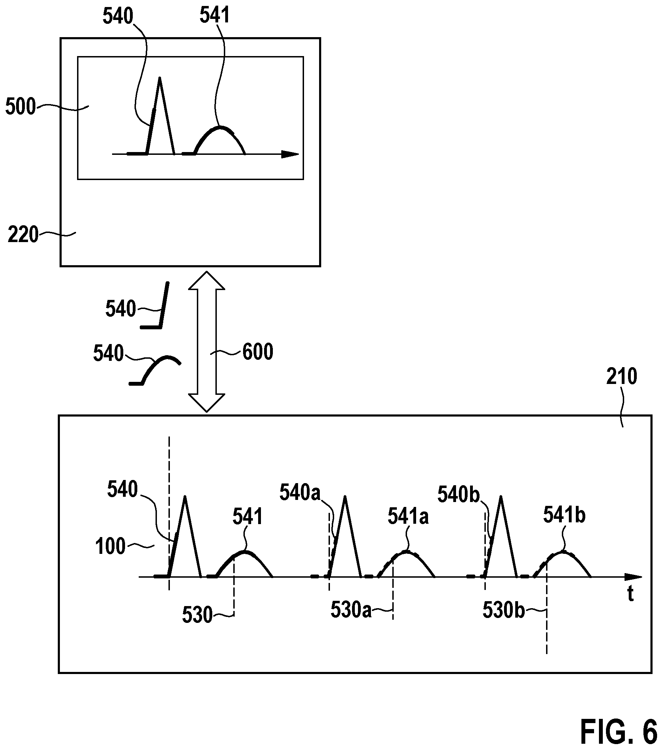

Figure 6 illustrates an embodiment in which a two-step signal analysis is performed. In a programming device 220 having an interactive display 500 for pattern identification, computing-intensive operations for identifying a first target pattern 540 and a second target pattern 541 are performed. The first target pattern 540 and the second target that a 541 are then transferred to a non-transvenous ICD 210 operatively coupled with the programming device 220. Here, less computing-intensive operations are performed to identify patterns within the electrocardiogram signal corresponding to the first target pattern 540 and/or the second target pattern 541. Reference is made to the explanations given with respect to Figure 5B. These less computing-intensive operations are, e.g., a correlation with convolution to identify the first target pattern 540 and/or the second target pattern 541 in signals representing subsequent cardiac events in real time. Due to a bidirectional communication 600 between the programming device 220 and the non-transvenous ICD 210, it is possible to transfer the sites of the electrocardiogram in which the first target pattern 540 and the second target pattern 541 have been identified to the programming device 220. The programming device can then double-check the identified patterns and/or visualize them on the interactive display 500.

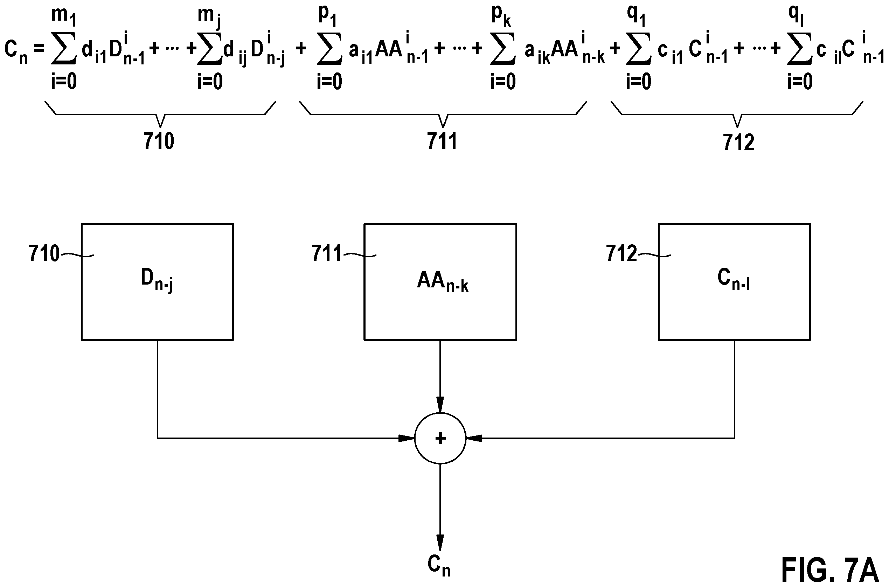

Figure 7A shows an exemplary embodiment of the function which is described as a Taylor series. A first sum term 710 relates to a plurality of "D" intervals between a T wave and a subsequent ventricular contraction event. Each of these intervals is multiplied with an individual coefficient $d_i$ (serving as third coefficient). At least one of the intervals is raised to the power of i.

The function further comprises a second sum term 711 relating to a plurality of "AA" intervals between two subsequent ventricular contraction events. Each of these "AA" intervals is multiplied with an individual coefficient $a_i$,

serving as first coefficient. At least one of the "AA" intervals is raised to the power of i.

The function further comprises a third sum term 712 relating to a plurality of "C" intervals between a ventricular contraction event and a subsequent T wave. Each of these "C" intervals is multiplied with an individual coefficient $c_i$, serving as second coefficient. At least one of the "C" intervals is raised to the power of i.

As visualized in Figure 7A, the first sum term 710, the second sum term 711, and the third sum term 712 are added to result in $C_n$, i.e., an interval measured from the most recent "A" event to the future T wave, thus being representative for the expected time of occurrence of the future T wave.

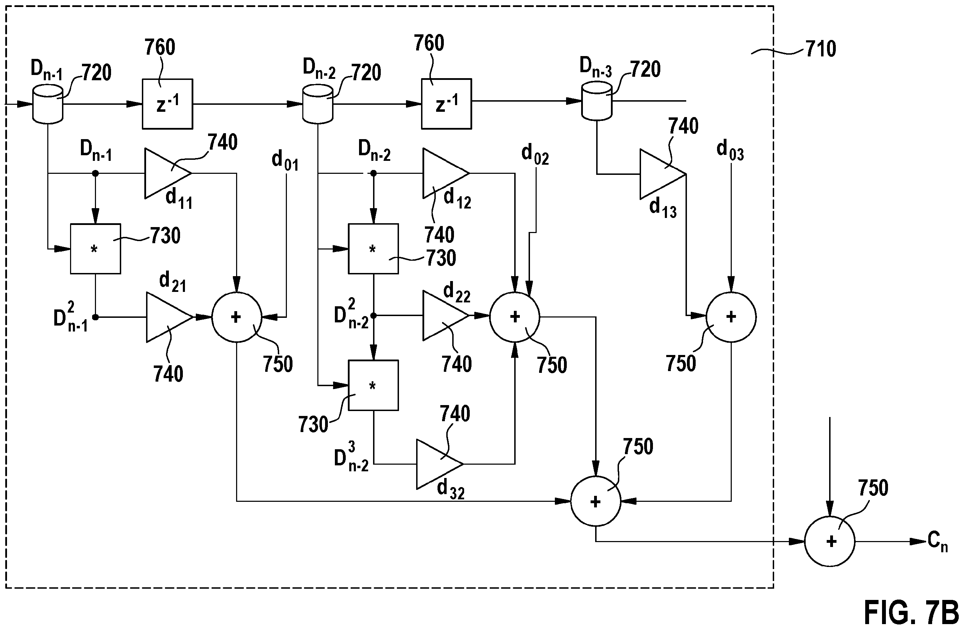

Figure 7B is a graphic representation of the first sum term 710 of the function illustrated in Figure 7A. First, the "D" interval $D_{n-1}$ is read from a storage 720. It is then further subjected to mathematical operations applied by a multiplayer 730, at least one amplifier 740, a summer 750, and a delay element 760. In this context, the exemplary embodiment illustrated in Figure 7B makes use of the following values of the parameters of the first sum term 710: $j = 3$, $m_1 = 2$, $m_2 = 3$, and $m_3 = 1$.

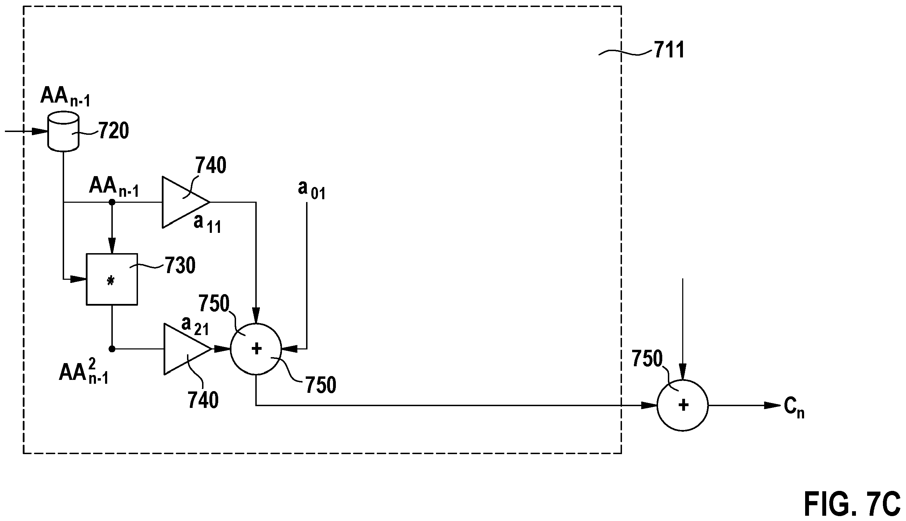

Figure 7C shows a similar visualization of the second sum term 711 of the function of Figure 7A. Here, the following values for the parameters of the second sum term 711 are used: $k = 1$, and $pi = 2$.

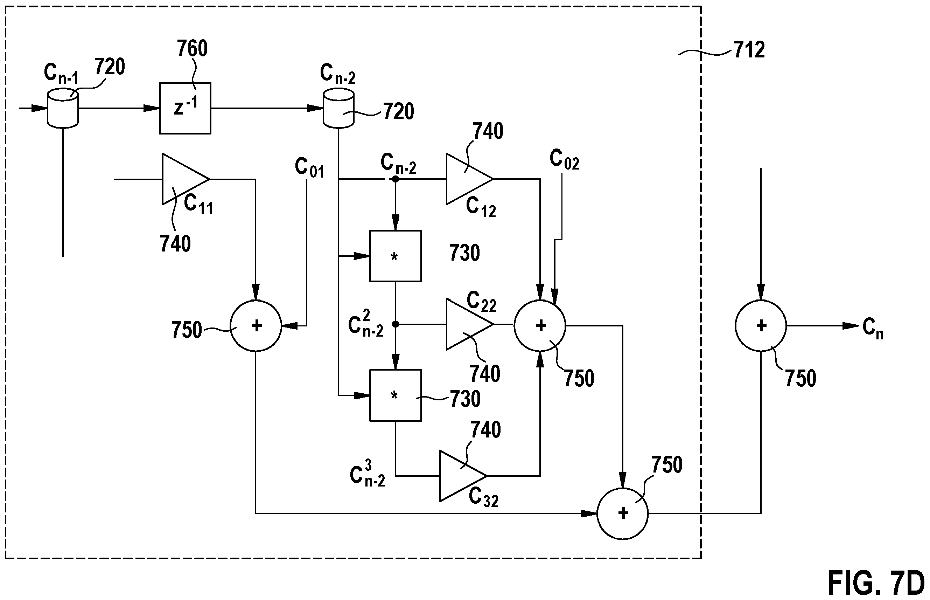

Likewise, Figure 7D graphically illustrates the third sum term 712 of the function illustrated in Figure 7A. For this embodiment, the following values for the parameter of the third sum term 712 are used: $l = 2$, $q_1 = 1$, and $q_2 = 3$.

The summer 750 displayed on the most right-hand side of Figures 7B, 7C, and 7D illustrates the summing of the first sum term 710, the second sum term 711 and the third sum term 712 to result in $C_n$, i.e., the value being representative for the expected time of occurrence of the future T wave.

## Claims

1. Arrangement (200) comprising

   a non-transvenous implantable cardioverter-defibrillator device (210) for stimulating a human or animal heart, the non-transvenous implantable cardioverter-defibrillator device (210) comprising a stimulation unit (270) for emitting stimulation pulses (140) externally to the heart; and
   a programming device (220) operatively coupled to the non-transvenous implantable cardioverter-defibrillator device (210);
   wherein the programming device (220) comprises a first processor (230) and a first memory unit, wherein the first memory unit comprises first computer-readable code that causes the first processor (230) to perform the following steps when executed on the first processor (230):

      obtaining, from the first memory unit, cardiac cycle data based on an analysis of first electrocardiogram signals (240) indicative of a natural cardiac activity of the heart;
      evaluating a function for predicting an expected time of occurrence of a future T wave ($B_n$) using the cardiac cycle data to determine at least one of a plurality of first coefficients, a plurality of second coefficients and a plurality of third coefficients;
      wherein the function comprises at least one of i) a sum of powers of a first interval (AA) between two subsequent ventricular contraction events (110), wherein each power of the first interval (AA) is multiplied by a first coefficient ($a_i$), ii) a sum of powers of a second interval (C) between a ventricular contraction event (110) and a subsequent T wave (130), wherein each power of the second interval (C) is multiplied by a second coefficient ($c_i$), and iii) a sum of powers of a third interval (D) between a T wave (130) and a subsequent ventricular contraction event (110), wherein each power of the third interval (D) is multiplied by a third coefficient ($d_i$),
      transmitting the first coefficients ($a_i$), the second coefficients ($c_i$) and/or the third coefficients ($d_i$) to the non-transvenous implantable cardioverter-defibrillator device (210);

   wherein the non-transvenous implantable cardioverter-defibrillator device (210) comprises a second processor (260) and a second memory unit, wherein the second memory unit comprises second computer-readable code that causes the second processor (260) to perform the following steps when executed on the second processor (260):

      receiving second electrocardiogram signals indicative of a natural cardiac activity of the heart;
      processing the second electrocardiogram signals to predict an expected time of occurrence of a future T wave ($B_n$),
      wherein the expected time of occurrence of the future T wave ($B_n$) is calculated by the function using the first coefficients ($a_i$), the second coefficients ($c_i$) and/or the third coefficients ($d_i$).

2. Arrangement (200) according to claim 1, **characterized in that** the first computer-readable code further causes the first processor (230) to perform the following steps when executed on the first processor (230): optimizing the first coefficients ($a_i$), the second coefficients ($c_i$) and/or the third coefficients ($d_i$) with an optimizing algorithm (232, 234, 235, 238, 239) to obtain optimized first coefficients (261), optimized second coefficients (261) and/or optimized third coefficients (261) before transmitting to the non-transvenous implantable cardioverter-defibrillator device (210).

3. Arrangement according to any one of claims 1 or 2, **characterized in that** at least one of the sum of powers of the first interval (AA) forms a series expansion in powers 0 to p of the first interval (AA), wherein the first coefficients ($a_i$) form expansion coefficients, the sum of powers of the second intercal (C) forms a series expansion in powers 0 to q of the second interval (C), wherein the second coefficients ($a_i$) form expansion coefficients, and the sum of powres of the third interval (D) forms a series expansion in powers 0 to m of the third interval (D), wherein the third coefficients ($d_i$) form expansion coefficients, wherein m, p, q are predetermined natural numbers.

4. Arrangement according to the preceding claim, **characterized in that** the series expansion in the first interval (AA), the series expansion in the second interval (C) and/or the series expansion in the third interval (D) forms at least a part of a Taylor series.

5. Arrangement according to any of the preceding claims, **characterized in that** the second computer-readable code causes the second processor (260) to generate at least one stimulation pulse emitted by the stimulation unit (270) during the expected time of occurrence of the future T wave ($B_n$).

6. Arrangement according to any of the preceding claims, **characterized in that** the second computer-readable code causes the second processor (260) to generate at least one stimulation pulse emitted by the stimulation unit (270) outside the expected time of occurrence of the future T wave ($B_n$).

7. Arrangement according to any one of claims 5 or 6, **characterized in that** the programming device triggers the second processor (260) of the non-transvenous implantable cardioverter-defibrillator (210) to generate the at least one stimulation pulse emitted by the stimulation unit (270).

8. Arrangement according to any of the preceding claims, **characterized in that** the absolute values of the optimized first coefficients (261), the absolute values of the optimized second coefficients (261), and/or the absolute values of the optimized third coefficients (261) decrease monotonically at least section-wise with increasing temporal distance between the future T wave ($B_n$) and the respective interval (AA; C; D) to which the the optimized first coefficients (261), of the optimized second coefficients (261), or of the optimized third coefficients (261) are assigned, respectively.

9. Arrangement according to any one of the preceding claims, **characterized in that** the cardiac cycle data comprises at least a length of the second interval (C) of a first cardiac cycle and at least one of a length of the first interval (AA) of the second cardiac cycle, a length of the second interval (C) of the second cardiac cycle, and a length of the third interval (D) of the second cardiac cycle, wherein the second cardiac cycle precedes the first cardiac cycle.

10. Arrangement according to any of the preceding claims, **characterized in that** the function corresponds to the following equation:

$$C_n = \sum_{i=0}^{m_1} d_{i1} D_{n-1}^i + \cdots + \sum_{i=0}^{m_j} d_{ij} D_{n-j}^i + \sum_{i=0}^{p_1} a_{i1} AA_{n-1}^i + \cdots + \sum_{i=0}^{p_k} a_{ik} AA_{n-k}^i$$

$$+ \sum_{i=0}^{q_1} c_{i1} C_{n-1}^i + \cdots + \sum_{i=0}^{q_l} c_{il} C_{n-l}^i$$

wherein

$C_n$ denotes the second interval in a first cardiac cycle;
$a_{i1}...a_{ik}$ denote the first coefficients or the optimized first coefficients;
$c_{i1}...c_{il}$ denote the second coefficients or the optimized second coefficients;

$d_{i1}...d_{ij}$ denote the third coefficients or the optimized third coefficients;

$AA_{n-1}...AA_{n-k}$ denote the first intervals in a second cardiac cycle and preceding cardiac cycles prior to the first cardiac cycle;

$C_{n-1}...C_{n-1}$ denote the second intervals in the second cardiac cycle and preceding cardiac cycles prior to the first cardiac cycle;

$D_{n-1}...D_{n-j}$ denote the third intervals of the second cardiac cycle and preceding cardiac cycles prior to the first cardiac cycle;

j, k, l denote independently from each other a number of cardiac cycles preceding the first cycle and may take values in a range from 1 to 10;

m, p, q denote the largest powers of the first, second and third intervals in the series expansions and are programmable independently from each other. m, p, q may take values in a range from 1 to 5, in particular from 1 to 3.

11. Arrangement according to any of the preceding claims, **characterized in that** the first computer-readable code causes the first processor (230) to perform the following step and/or **in that** the second computer-readable code causes the second processor (260) to perform the following step: performing pattern-identification calculations resulting in pattern-recognition information (540, 541) and storing the pattern-recognition information (540, 541) in the first or the second memory unit, respectively.

12. Arrangement according to the preceding claim, **characterized in that** the first computer-readable code causes the first processor (230) to perform the following step and/or **in that** the second computer-readable code causes the second processor (260) to perform the following step: applying a pattern-tracking algorithm on the electrocardiogram signals (240) to identify cardiac events in the first or the second electrocardiogram signals (240), in particular by correlating signals of the electrocardiogram signals (240) with the stored pattern-recognition information (540, 541), wherein correlating signals of the electrocardiogram signals (240), in particular, involves a convolution of the electrocardiogram signals (240).

13. Arrangement according to any of the preceding claims, **characterized in that** the first computer-readable code causes the first processor (230) to optimize the first coefficients ($a_i$), the second coefficients ($c_i$) and/or the third coefficients ($d_i$) by a) measuring a factual time (237) of occurrence of the future T wave ($B_n$), b) determining a difference between the factual time (237) of occurrence of the future T wave ($B_n$) and the expected time (236) of occurrence of the future T wave ($B_n$), and c) iteratively reducing (232, 234, 235, 238, 239) the difference until it falls below a predeterminable threshold, wherein the predeterminable threshold lies in a range of from 10 ms to 100 ms.

14. Processing method for determining an expected time of occurrence of a future T wave ($B_n$) in a cardiac cycle of a human or animal heart,

wherein the method is carried out by a processor of a medical device and comprises the following steps:

obtaining cardiac cycle data based on an analysis of first electrocardiogram signals (240) indicative of a natural cardiac activity of the heart;

evaluating a function for predicting an expected time of occurrence of a future T wave ($B_n$) using the cardiac cycle data to determine at least one of a plurality of first coefficients ($a_i$), a plurality of second coefficients ($c_i$) and a plurality of third coefficients ($d_i$);

wherein the function comprises at least one of i) a sum of powers of a first interval (AA) between two subsequent ventricular contraction events (110), wherein each power of the first interval (AA) is multiplied by a first coefficient ($a_i$), ii) a sum of powers of a second interval (C) between a ventricular contraction event (110) and a subsequent T wave (130), wherein each power of the second interval (C) is multiplied by a second coefficient ($c_i$), and iii) a sum of powers of a third interval (D) between a T wave (130) and a subsequent ventricular contraction event (110), wherein each power of the third interval (D) is multiplied by a third coefficient ($d_i$),

receiving second electrocardiogram signals indicative of a natural cardiac activity of the heart;

processing the second electrocardiogram signals to predict an expected time of occurrence of a future T wave ($B_n$),

wherein the expected time of occurrence of the future T wave ($B_n$) is calculated by the function using the first coefficients ($a_i$), the second coefficients ($c_i$) and/or the third coefficients ($d_i$).

15. Non-transvenous implantable cardioverter-defibrillator device (210) for stimulating a human or animal heart, the non-transvenous implantable cardioverter-defibrillator device (210) comprising a stimulation unit (270) for emitting stimulation pulses (140) externally to the heart, , a processor (230) and a memory unit, wherein the memory unit comprises computer-readable code that causes the processor (230) to perform the following steps when executed on the processor (230):

obtaining, from the memory unit, cardiac cycle data based on an analysis of first electrocardiogram signals (240) indicative of a natural cardiac activity of the heart;

evaluating a function for predicting an expected time of occurrence of a future T wave ($B_n$) using the cardiac cycle data to determine at least one of a plurality of first coefficients ($a_i$), second coefficients ($c_i$) and/or third coefficients ($d_i$);

wherein the function comprises at least one of i) a sum of powers of a first interval (AA) between two subsequent ventricular contraction events (110), wherein each power of the first interval (AA) is multiplied by a first coefficient ($a_i$), ii) a sum of powers of a second interval (C) between a ventricular contraction event (110) and a subsequent T wave (130), wherein each power of the second interval (C) is multiplied by a second coefficient ($c_i$), and iii) a sum of powers of a third interval (D) between a T wave (130) and a subsequent ventricular contraction event (110), wherein each power of the third interval (D) is multiplied by a third coefficient ($d_i$),

receiving second electrocardiogram signals indicative of a natural cardiac activity of the heart;

processing the second electrocardiogram signals to predict an expected time of occurrence of a future T wave ($B_n$),

wherein the expected time of occurrence of the future T wave ($B_n$) is calculated by the function using the first coefficients ($a_i$), the second coefficients ($c_i$) and/or the third coefficients ($d_i$).

FIG. 1A

FIG. 1B

FIG. 2A

FIG. 2B

FIG. 2C

FIG. 3

FIG. 4

530    520a

500

520
510

220

**FIG. 5A**

540    540a    540b
541    541a    541b
100

530    530a    530b

t

**FIG. 5B**

FIG. 6

$$C_n = \overbrace{\sum_{i=0}^{m_1} d_{i1}D_{n-1}^i + \cdots + \sum_{i=0}^{m_j} d_{ij}D_{n-j}^i}^{710} + \overbrace{\sum_{i=0}^{p_1} a_{i1}AA_{n-1}^i + \cdots + \sum_{i=0}^{p_k} a_{ik}AA_{n-k}^i}^{711} + \overbrace{\sum_{i=0}^{q_1} c_{i1}C_{n-1}^i + \cdots + \sum_{i=0}^{q_l} c_{il}C_{n-1}^i}^{712}$$

FIG. 7A

FIG. 7B

EP 4 620 517 A1

FIG. 7C

EP 4 620 517 A1

FIG. 7D

Europäisches
Patentamt
European
Patent Office
Office européen
des brevets

# EUROPEAN SEARCH REPORT

**Application Number**

EP 24 16 4308

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2023/172961 A1 (ZOLL MEDICAL CORP [US]) 14 September 2023 (2023-09-14) * abstract; figures 3, 4 * * paragraph [0067] - paragraph [0069] * | 1-15 | INV. A61N1/39 |
| A | US 2008/033494 A1 (SWERDLOW CHARLES [US]) 7 February 2008 (2008-02-07) * the whole document * | 1-15 | |
| A | US 8 831 722 B2 (SWERDLOW CHARLES [US]; IMPERCEPTION INC [US]) 9 September 2014 (2014-09-09) * the whole document * | 1-15 | |

**TECHNICAL FIELDS SEARCHED (IPC)**

A61N

The present search report has been drawn up for all claims

| Place of search | Date of completion of the search | Examiner |
|---|---|---|
| Munich | 13 August 2024 | Wetzig, Thomas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 16 4308

This annex lists the patent family members relating to the patent documents cited in the above-mentioned European search report.
The members are as contained in the European Patent Office EDP file on
The European Patent Office is in no way liable for these particulars which are merely given for the purpose of information.

13-08-2024

| Patent document cited in search report | Publication date | Patent family member(s) | Publication date |
|---|---|---|---|
| WO 2023172961 A1 | 14-09-2023 | NONE | |
| US 2008033494 A1 | 07-02-2008 | US 2008033494 A1<br>US 2008051841 A1 | 07-02-2008<br>28-02-2008 |
| US 8831722 B2 | 09-09-2014 | NONE | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20030195569 A1

- US 7386342 B

**Non-patent literature cited in the description**

- **BAROLD, H. S.** ; **WHARTON, J. M.** Ventricular fibrillation resulting from synchronized internal atrial defibrillation in a patient with ventricular preexcitation. *Journal of Cardiovascular Electrophysiology*, 1997, vol. 8 (4), 436-440

- **WYLIE JR, J. V.** ; **ESSEBAG, V.** ; **REYNOLDS, M. R.** ; **JOSEPHSON, M. E.** Inducibility of Atrial Fibrillation with a Synchronized External Low Energy Shock Post-Pulmonary Vein Isolation Predicts Recurrent Atrial Fibrillation. *Journal of Cardiovascular Electrophysiology*, 2009, vol. 20 (1), 29-36